# FASCICULE DE BREVET EUROPEEN

(11) **EP 1 041 956 B1**
(45) Date de publication et mention de la délivrance du brevet: **19.10.2005**
(21) Numéro de dépôt: 99900518.4
(22) Date de dépôt: 12.01.1999
(51) Int. Cl.: A61K 7/13

(54) **COMPOSITION DE TEINTURE D'OXYDATION DES FIBRES KERATINIQUES CONTENANT UNE LACCASE ET PROCEDE DE TEINTURE METTANT EN OEUVRE CETTE COMPOSITION**
OXIDATIONSFÄRBEMITTEL FÜR KERATINFASERN, DAS EINE LACCASE ENTHÄLT, UND VERFAHREN ZUR FÄRBUNG MIT DIESEM MITTEL
MIXTURE FOR THE OXIDATION TINTING OF KERATIN FIBRES CONTAINING A LACCASE AND TINTING METHOD USING SAID MIXTURE

(30) Priorité: 13.01.1998 FR 9800252
(43) Date de publication de la demande: 11.10.2000
(73) Titulaire: L'OREAL, 75008 Paris (FR)
(72) Inventeur: LANG, Gérard, F-95390 Saint Prix (FR); COTTERET, Jean, F-78480 Verneuil/Seine (FR)
(74) Mandataire: Miszputen, Laurent
(86) Numéro de dépôt international: PCT/FR1999/000037
(87) Numéro de publication internationale: WO 1999/036044

(56) Documents cités:
- EP-A- 0 504 005
- EP-A- 0 557 203
- EP-A- 0 667 143
- EP-A- 0 769 958
- WO-A-97/39727
- FR-A- 2 694 018
- US-A- 5 514 188

## Description

La présente invention a trait à une composition tinctoriale des fibres kératiniques comprenant au moins une enzyme de type laccase , au moins un solvant choisi parmi certains polyols ou leurs éthers ou polyéthers, et au moins un colorant d'oxydation, ainsi que ses utilisations pour la teinture des fibres kératiniques en particulier des cheveux humains.

Il est connu de teindre les fibres kératiniques et en particulier les cheveux humains avec des compositions tinctoriales contenant des précurseurs de coloration d'oxydation, en particulier des ortho ou paraphénylènediamines, des ortho ou paraaminophénols, des bases hétérocycliques, appelés généralement bases d'oxydation. Les précurseurs de coloration d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

La coloration d'oxydation des fibres kératiniques est généralement réalisée en milieu alcalin, en présence de peroxyde d'hydrogène. Toutefois, l'utilisation des milieux alcalins en présence de peroxyde d'hydrogène présentent pour inconvénient d'entraîner une dégradation non négligeable des fibres, ainsi qu'une décoloration des fibres kératiniques qui n'est pas toujours souhaitable.

La coloration d'oxydation des fibres kératiniques peut également être réalisée à l'aide de systèmes oxydants différents du peroxyde d'hydrogène tels que des systèmes enzymatiques. Ainsi il a déjà été proposé dans le brevet US 3251742, les demandes de brevet FR-A-2 112 549, FR-A-2 694 018, EP-A-0 504 005, WO95/07988, WO95/33836, WO95/33837, WO96/00290, WO97/19998 et WO97/19999 de teindre les fibres kératiniques avec des compositions comprenant au moins un colorant d'oxydation en association avec des enzymes du type laccase ; lesdites compositions étant mises en contact avec l'oxygène de l'air. Ces formulations de teinture, bien qu'étant mises en oeuvre dans des conditions n'entraînant pas une dégradation des fibres kératiniques comparable à celle engendrée par les teintures réalisées en présence de peroxyde d'hydrogène, conduisent à des colorations encore insuffisantes à la fois sur le plan de l'homogénéité de la couleur répartie le long de la fibre (« unisson »), sur le plan de la chromaticité (luminosité) et de la puissance tinctoriale.

La présente invention a pour but de résoudre les problèmes évoqués ci-dessus.

La Demanderesse a découvert de façon surprenante de nouvelles compositions contenant au moins comme système oxydant une enzyme du type laccase et au moins un solvant particulier que l'on définira plus en détail ci-dessous, pouvant constituer en présence de colorant(s) d'oxydation (base d'oxydation et/ou coupleurs), des formulations de teinture prêtes à l'emploi conduisant à des colorations plus homogènes, plus puissantes et plus chromatiques sans engendrer de dégradation significative, ni de décoloration des fibres kératiniques, peu sélectives et résistant bien aux diverses agressions que peuvent subir les cheveux

Ces découvertes sont à la base de la présente invention.

La présente invention a donc pour premier objet une composition prête à l'emploi destinée à la teinture des fibres kératiniques humaines et plus particulièrement les cheveux humains, comprenant dans un support approprié pour la teinture des fibres kératiniques :
(a) au moins une enzyme du type laccase ;
(b) au moins un solvant choisi parmi
   - les αω-diols ramifiés ou non ramifiés en C₃-C₈ ;
   - les diols 1,2 - 1,4 - 1,5 - 2,3 - 2,4 - 2,5 - 2,6 - 2,7 - 2,8 - 3,4 - 3,5 - 3,6 en C₅-C₈ ramifiés ou non ramifiés ;
   - les diols 1,3 en C₆-C₈ ramifiés ou non ramifiés ;
   - les triols en C₄-C₈ ;
   - le diéthylène glycol et le dipropylène glycol ;
   - les monoalkyléthers en C₃-C₈ de glycols en C₃-C₉ ; monoalkyléthers C₅-C₉ d'éthylène glycol ; monoalkyléthers en C₁-C₂ de glycols en C₅-C₉ ; monoalkyléthers en C₂ des 1,2 - 1,3 - 1,4 - 2,3- butanediols ; les monoalkyléthers de glycols à nombre total d'atomes de carbone égal à 5 ;
   - les dialkyléthers en C₁-C₉ de glycols en C₂-C₉ ;
   - les phényl éthers de glycols en C₃-C₉ ;
(c) au moins un colorant d'oxydation.

La ou les laccases utilisées dans la composition tinctoriale prête à l'emploi conforme à l'invention peuvent notamment être choisies parmi les laccases d'origine végétale, d'origine animale, d'origine fongique (levures, moisissures, champignons) ou d'origine bactérienne, les organismes d'origine pouvant être mono ou pluricellulaires. Elles peuvent être obtenues par biotechnologie.

Parmi les laccases d'origine végétale utilisables selon l'invention , on peut citer les laccases produites par des végétaux effectuant la synthèse chlorophyllienne telles qu'indiquées dans la demande FR-A-2 694 018 comme celles que l'on retrouve dans les extraits des Anacardiacées tels que par exemple les extraits de Magnifera indica, Schinus molle ou Pleiogynium timoriense, dans les extraits des Podocarpacées , de Rosmarinus off., de Solanum tuberosum, d'Iris sp., de Coffea sp. , de Daucus carrota, de Vinca minor, Persea americana, Catharenthus roseus, Musa sp., Malus pumila, Gingko biloba, Monotropa hypopithys (sucepin), Aesculus sp., Acer pseudoplatanus, Prunus persica, Pistacia palaestina.

Parmi les laccases d'origine fongique éventuellement obtenues par biotechnologie utilisables selon l'invention, on peut citer la ou les laccases issues de Polyporus versicolor, de Rhizoctonia praticola et de Rhus vernicifera comme indiquées dans les demandes FR-A-2 112 549 et EP-A-504005 ; celles décrites dans les demandes de brevet WO95/07988, WO95/33836, WO95/33837, WO96/00290, WO97/19998 et WO97/19999, dont le contenu fait partie intégrante de la présente description comme par exemple celles issues de Scytalidium, de Polyporus pinsitus, de Myceliophtora thermophila, de Rhizoctonia solani, de Pyricularia orizae, ou leurs variantes. On peut aussi citer celles issues de Tramates versicolor, de Fomes fomentarius, de Chaetomium thermophile, de Neurospora crassa, de Coriolus versicol, de Botrytis cinerea, de Rigidoporus lignosus, de Phellinus noxius, de Pleurotus ostreatus, d'Aspergillus nidulans, de Podospora anserina, d'Agaricus bisporus, de Ganoderma lucidum, de Glomerella cingulata, de Lactarius piperatus, de Russula delica, d'Heterobasidion annosum, de Thelephora terrestris, de Cladosporium cladosporioides, de Cerrena unicolor, de Coriolus hirsutus, de Ceriporiopsis subvermispora, de Coprinus cinereus, de Panaeolus papilionaceus, de Panaeolus sphinctrinus, de Schizophyllum commune, de Dichomitius squalens et de leurs variantes.

On choisira plus préférentiellement les laccases d'origine fongique éventuellement obtenues par biotechnologie.

L'activité enzymatique des laccases de l'invention ayant la syringaldazine parmi leurs substrats peut être définie à partir de l'oxydation de la syringaldazine en condition aérobie. L'unité lacu correspond à la quantité d'enzyme catalysant la conversion de 1mmole de syringaldazine par minute à pH 5,5 à 30°C. L'unité u correspond à la quantité d'enzyme produisant un delta d'absorbance à 530 nm de 0,001 par minute en utilisant la syringaldazine comme substrat, à 30°C et à pH 6,5.
L'activité enzymatique des laccases de l'invention peut aussi être définie à partir de l'oxydation de la paraphénylènediamine. L'unité ulac correspond à la quantité d'enzyme produisant un delta d'absorbance à 496,5nm de 0,001 par minute en utilisant la paraphénylènediamine comme substrat (64 mM) à 30°C et à pH 5.
Selon l'invention, on préfère déterminer l'activité enzymatique en unités ulac.
Les quantités de laccase utilisées dans les compositions de l'invention varieront en fonction de la nature de la laccase choisie. De façon préférentielle, elles varieront de 0,5 à 2000 lacu, ou de 1000 à 4.10⁷ unités u, ou de 20 à 2.10⁶ unités ulac pour 100g de composition.

Par polyol, on désigne au sens de l'invention, un composé de type alkyle, linéaire, ramifié ou cyclique, saturé ou insaturé, portant au moins deux fonctions -OH sur la chaîne alkyle, ainsi que les polymères (polyéthers) de ces composés alkyle polyhydroxylés.

Pour les αω-diols ramifiés ou non ramifiés en C₃-C₈, αω s'entend, quand il s'agit de composés ramifiés, par rapport à l'enchaînement carboné le plus long.

Parmi ces composés, on peut citer par exemple, le 1,3 propane-diol ; le 1,4-butanediol ; le 1,5-pentanediol ; le 1,6-hexanediol ; le néopentylglycol (ou 2,3-diméthyl-1,3-propanediol).
Parmi les diols 1,2 - 1,4 - 1,5 - 2,3 - 2,4 - 2,5 - 2,6 - 2,7 - 2,8 - 3,4 - 3,5 - 3,6 en C₅-C₈ ramifiés ou non ramifiés, on peut citer, le 2,5-hexanediol ; le 2,4-pentanediol (ou amylèneglycol) ; le 2-méthyl-2,4-pentanediol (ou hexylèneglycol) ; le 2,3-diméthyl-2,3-butanediol (ou pinacol).
Parmi les triols en C₄-C₈ on peut notamment citer le 1,2,4-butanetriol et le 1,2,6-hexanetriol.
Parmi les monoalkyléthers de glycols à nombre total d'atomes de carbone égal à 5, on peut citer le monoéthyléther de propylèneglycol .
Parmi les monoalkyléthers en C₃-C₈ de glycols en C₃-C₉ on peut citer le monobutyléther de propylèneglycol .
Parmi les monoalkyléthers C₅-C₉ d'éthylène glycol on peut citer le monohexyléther d'éthylèneglycol .
Parmi les monoalkyléthers en C₁-C₂ de glycols en C₅-C₉ on peut citer les monométhyléther de dipropylèneglycol, le monométhyléther d'isoprèneglycol , le monométhyléther de tripropylèneglycol .
Parmi les monoalkyléthers en C₂ des 1,2 - 1,3 - 1,4 - 2,3- butanediols on peut citer le monométhyléther de 2,3-butanediol.
Parmi les dialkyléthers en C₁-C₉ de glycols en C₂-C₉ , on peut citer le dibutyléther de propylèneglycol .
Parmi les phényl éthers de glycols en C₃-C₉ , on peut citer notamment le monophényléther de propylèneglycol et le monophényléther de diéthylèneglycol.

Dans la présente invention, on préfère utiliser, à titre de solvant :
- les αω-diols ramifiés ou non ramifiés en C₃-C₈ ;
- le diéthylène glycol et le dipropylène glycol ;
- les monoalkyléthers en C₃-C₈ de glycols en C₃-C₉ ;
- les monoalkyléthers en C₁-C₂ de glycols en C₅-C₉.

Les polyols ou leurs éthers ou polyéthers décrits ci-avant sont présents dans la composition tinctoriale conforme à l'invention dans des proportions généralement comprises entre 0,1 et 40% en poids, et encore plus particulièrement de 0,5 à 20% en poids par rapport au poids total de la composition.

La nature de la ou des bases d'oxydation et/ou des coupleurs utilisés dans la composition tinctoriale prête à l'emploi n'est pas critique.
Les bases d'oxydation peuvent notamment être choisies parmi les paraphénylènediamines, les bases doubles, les para-aminophénols, les ortho aminophénols et les bases d'oxydation hétérocycliques.

Parmi les paraphénylènediamines utilisables à titre de base d'oxydation dans les composition tinctoriales conformes à l'invention, on peut notamment citer les composés de formule (I) suivante et leurs sels d'addition avec un acide : 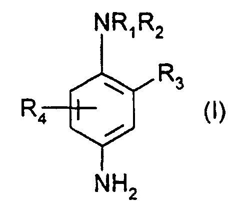 dans laquelle :
- R₁ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄), alkyle en C₁-C₄ substitué par un groupement azoté, phényle ou 4'-aminophényle ;
- R₂ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄) ou alkyle en C₁-C₄ substitué par un groupement azoté ;
- R₃ représente un atome d'hydrogène, un atome d'halogène tel qu'un atome de chlore, de brome, d'iode ou de fluor, un radical alkyle en C₁-C₄, monohydroxyalkyle en C₁-C₄, hydroxyalcoxy en C₁-C₄, acétylaminoalcoxy en C₁-C₄, mésylaminoalcoxy en C₁-C₄ ou carbamoylaminoalcoxy en C₁-C₄,
- R₄ représente un atome d'hydrogène, d'halogène ou un radical alkyle en C₁-C₄.

Parmi les groupements azotés de la formule (I) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les paraphénylènediamines de formule (I) ci-dessus, on peut plus particulièrement citer la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthylparaphénylènediamine, la 2-fluoro-para-phénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl para-phénylènediamine, la 2-β-hydroxyéthyloxy paraphénylènediamine, la 2-β-acétylamino-éthyloxy paraphénylènediamine, la N-(β-méthoxyéthyl) paraphénylènediamine, et leurs sels d'addition avec un acide.

Parmi les paraphénylènediamines de formule (I) ci-dessus, on préfère tout particulièrement la paraphénylènediamine, la paratoluylènediamine, la 2-isopropyl paraphénylènediamine, la 2-β-hydroxyéthyl paraphénylènediamine, la 2-β-hydroxy-éthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide.

Selon l'invention, on entend par bases doubles, les composés comportant au moins deux noyaux aromatiques sur lesquels sont portés des groupements amino et/ou hydroxyle.

Parmi les bases doubles utilisables à titre de bases d'oxydation dans les compositions tinctoriales conformes à l'invention, on peut notamment citer les composés répondant à la formule (II) suivante, et leurs sels d'addition avec un acide : 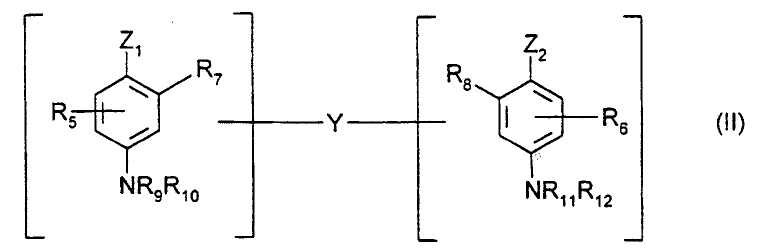 dans laquelle :
- Z₁ et Z₂, identiques ou différents, représentent un radical hydroxyle ou -NH₂ pouvant être substitué par un radical alkyle en C₁-C₄ ou par un bras de liaison Y ;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₆ ;
- R₅ et R₆ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison Y ;
- R₇, R₈, R₉, R₁₀ R₁₁ et R₁₂, identiques ou différents, représentent un atome d'hydrogène, un bras de liaison Y ou un radical alkyle en C₁-C₄ ; étant entendu que les composés de formule (II) ne comportent qu'un seul bras de liaison Y par molécule.

Parmi les groupements azotés de la formule (II) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les bases doubles de formules (II) ci-dessus, on peut plus particulièrement citer le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino-phénoxy)-3,5-dioxaoctane, et leurs sels d'addition avec un acide.

Parmi ces bases doubles de formule (II), le N,N'-bis-(β-hydroxyéthyl) N,N'-bis-(4'-amino-phényl) 1,3-diamino propanol, le 1,8-bis-(2,5-diaminophénoxy)-3,5-dioxaoctane ou l'un de leurs sels d'addition avec un acide sont particulièrement préférés.

Parmi les para-aminophénols utilisables à titre de bases d'oxydation dans les compositions tinctoriales conformes à l'invention, on peut notamment citer les composés répondant à la formule (III) suivante, et leurs sels d'addition avec un acide : 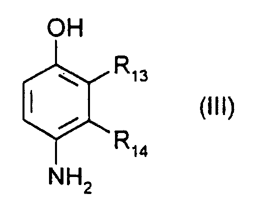 dans laquelle :
- R₁₃ représente un atome d'hydrogène ou d'halogène, un radical alkyle en C₁-C₄, mono-hydroxyalkyle en C₁-C₄, alcoxy(C₁-C₄)alkyle(C₁-C₄), aminoalkyle en C₁-C₄ ou hydroxyalkyl(C₁-C₄)aminoalkyle en C₁-C₄,
- R₁₄ représente un atome d'hydrogène ou d'halogène, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄),
étant entendu qu'au moins un des radicaux R₁₃ ou R₁₄ représente un atome d'hydrogène.

Parmi les para-aminophénols de formule (III) ci-dessus, on peut plus particulièrement citer le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les orthoaminophénols utilisables à titre de bases d'oxydation dans les compositions tinctoriales conformes à l'invention, on peut plus particulièrement citer le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques utilisables à titre de bases d'oxydation dans les compositions tinctoriales conformes à l'invention, on peut plus particulièrement citer les dérivés pyridiniques, les dérivés pyrimidiniques, les dérivés pyrazoliques, les dérivés pyrazolo-pyrimidiniques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

Parmi les dérivés pyrimidiniques, on peut plus particulièrement citer les composés décrits par exemple dans les brevets allemand DE 2359399 ou japonais JP88-169571 et JP 91-33 495 ou demandes de brevet WO 96/15765, comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et leurs sels d'addition avec un acide.

Parmi les dérivés pyrazoliques, on peut plus particulièrement citer les composés décrits dans les brevets DE 3843 892, DE 4 133 957 et demandes de brevet WO 94/08969, WO 94/08970, FR-A-2 733 749 et DE 195 43 988 comme le 4,5-diamino 1-méthyl pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

Parmi les dérivés pyrazolo-pyrimidiniques, on peut plus particulièrement citer les pyrazolo-[1,5-a]-pyrimidines de formule (IV) suivante, leurs sels d'addition avec un acide ou avec une base et leurs formes tautomères, lorsqu'il existe un équilibre tautomérique : 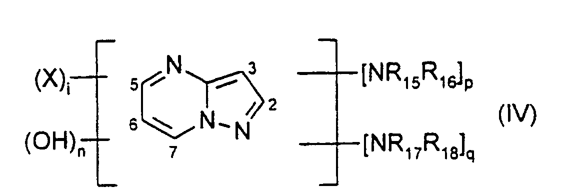 dans laquelle :
- R₁₅, R₁₆, R₁₇ et R₁₈, identiques ou différents désignent, un atome d'hydrogène, un radical alkyle en C₁-C₄, un radical aryle, un radical hydroxyalkyle en C₁-C₄, un radical polyhydroxyalkyle en C₂-C₄, un radical (C₁-C₄)alcoxy alkyle en C₁-C₄, un radical aminoalkyle en C₁-C₄ (l'amine pouvant être protégée par un radical acétyle, uréido ou sulfonyle), un radical (C₁-C₄)alkylamino alkyle en C₁-C₄, un radical di-[(C₁-C₄)alkyl] amino alkyle en C₁-C₄ (les radicaux dialkyles pouvant former un cycle carboné ou un hétérocycle à 5 ou 6 chaînons), un radical hydroxy(C₁-C₄)alkyl- ou di-[hydroxy(C₁-C₄) alkyl]-amino alkyle en C₁-C₄ ;
- les radicaux X désignent , identiques ou différents, un atome d'hydrogène, un radical alkyle en C₁-C₄, un radical aryle, un radical hydroxyalkyle en C₁-C₄, un radical polyhydroxyalkyle en C₂-C₄, un radical amino alkyle en C₁-C₄, un radical (C₁-C₄)alkyl amino alkyle en C₁-C₄, un radical di-[(C₁-C₄)alkyl] amino alkyle en C₁-C₄ (les dialkyles pouvant former un cycle carboné ou un hétérocycle à 5 ou 6 chaînons), un radical hydroxy(C₁-C₄)alkyl ou di-[hydroxy(C₁-C₄)alkyl]amino alkyle en C₁-C₄, un radical amino, un radical (C₁-C₄)alkyl- ou di-[(C₁-C₄)alkyl]-amino ; un atome d'halogène, un groupe acide carboxylique, un groupe acide sulfonique ;
- i vaut 0, 1, 2 ou 3 ;
- p vaut 0 ou 1 ;
- q vaut 0 ou 1 ;
- n vaut 0 ou 1 ;
sous réserve que :
- la somme p + q est différente de 0 ;
- lorsque p + q est égal à 2, alors n vaut 0 et les groupes NR₁₅R₁₆ et NR₁₇R₁₈ occupent les positions (2,3) ; (5,6) ; (6,7) ; (3,5) ou (3,7) ;
- lorsque p + q est égal à 1 alors n vaut 1 et le groupe NR₁₅R₁₆ (ou NR₁₇R₁₈) et le groupe OH occupent les positions (2,3) ; (5,6) ; (6,7) ; (3,5) ou (3,7) ;

Lorsque les pyrazolo-[1,5-a]-pyrimidines de formule (IV) ci-dessus sont telles qu'elles comportent un groupe hydroxyle sur l'une des positions 2, 5 ou 7 en α d'un atome d'azote, il existe un équilibre tautomérique représenté par exemple par le schéma suivant :

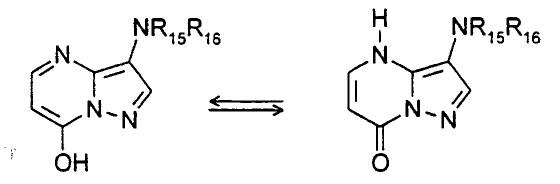

Parmi les pyrazolo-[1,5-a]-pyrimidines de formule (IV) ci-dessus on peut notamment citer :
- la pyrazolo-[1,5-a]-pyrimidine-3,7-diamine ;
- la 2,5-diméthyl pyrazolo-[1,5-a]-pyrimidine-3,7-diamine ;
- la pyrazolo-[1,5-a]-pyrimidine-3,5-diamine ;
- la 2,7-diméthyl pyrazolo-[1,5-a]-pyrimidine-3,5-diamine ;
- le 3-amino pyrazolo-[1,5-a]-pyrimidin-7-ol
- le 3-amino pyrazolo-[1,5-a]-pyrimidin-5-ol
- le 2-(3-amino pyrazolo-[1,5-a]-pyrimidin-7-ylamino)-éthanol
- le 2-(7-amino pyrazolo-[1,5-a]-pyrimidin-3-ylamino)-éthanol
- le 2-[(3-Amino-pyrazolo[1,5-a]pyrimidin-7-yl)-(2-hydroxy-éthyl)-amino]-éthanol
- le 2-[(7-Amino-pyrazolo[1,5-a]pyrimidin-3-yl)-(2-hydroxy-éthyl)-amino]-éthanol
- la 5,6-diméthyl pyrazolo-[1,5-a]-pyrimidine-3,7-diamine ;
- la 2,6-diméthyl pyrazolo-[1,5-a]-pyrimidine-3,7-diamine ;
- la 2, 5, N 7, N 7-tetraméthyl pyrazolo-[1,5-a]-pyrimidine-3,7-diamine ;
et leurs sels d'addition et leurs formes tautomères, lorsqu'il existe un équilibre tautomérique.

Les pyrazolo-[1,5-a]-pyrimidines de formule (IV) ci-dessus peuvent être préparées par cyclisation à partir d'un aminopyrazole selon les synthèses décrites dans les références suivantes :
- EP 628559 BEIERSDORF-LILLY
- R. Vishdu, H. Navedul, Indian J. Chem., 34b (6), 514, 1995.
- N.S. Ibrahim, K.U. Sadek, F.A. Abdel-Al, Arch. Pharm., 320, 240, 1987.
- R.H. Springer, M.B. Scholten, D.E. O'Brien, T. Novinson, J.P. Miller, R.K. Robins, J. Med. Chem., 25, 235, 1982.
- T. Novinson, R.K. Robins, T.R. Matthews, J. Med. Chem., 20, 296, 1977.
- US 3907799 ICN PHARMACEUTICALS

Les pyrazolo-[1,5-a]-pyrimidines de formule (IV) ci-dessus peuvent également être préparées par cyclisation à partir d'hydrazine selon les synthèses décrites dans les références suivantes :
- A. McKillop et R.J. Kobilecki, Heterocycles, 6(9), 1355, 1977.
- E. Alcade, J. De Mendoza, J.M. Marcia-Marquina, C. Almera, J. Elguero, J. Heterocyclic Chem., 11 (3), 423, 1974.
- K. Saito, I. Hori, M. Higarashi, H. Midorikawa, Bull. Chem. Soc. Japan, 47(2), 476, 1974.

La ou les bases d'oxydation conformes à l'invention représentent de préférence de 0,0005 à 12 % en poids environ du poids total de la composition tinctoriale prête à l'emploi, et encore plus préférentiellement de 0,005 à 6 % en poids environ de ce poids.

Le ou les coupleurs pouvant être utilisés dans la composition tinctoriale prête à l'emploi conforme à l'invention sont ceux classiquement utilisés dans les compositions de teinture d'oxydation, c'est-à-dire des métaphénylènediamines, des métaaminophénols et des métadiphénols, les dérivés mono- ou polyhydroxylés du naphtalène, le sésamol et ses dérivés et des composés hétérocycliques tels que par exemple les dérivés indoliques, les dérivés indoliniques, les dérivés de benzimidazole, les dérivés de benzomorpholine, les dérivés de sésamol, les dérivés pyrazolo-azoliques, les dérivés pyrrolo-azoliques, les dérivés imidazolo-azoliques, les dérivés pyrazolo-pyrimidiniques, les dérivés de pyrazolin-3,5-diones, les dérivés pyrrolo-[3,2-d]-oxazoliques, les dérivés pyrazolo-[3,4-d]-thiazoliques, les dérivés S-oxyde-thiazolo-azoliques, les dérivés S,S-dioxyde-thiazolo-azoliques, et leurs sels d'addition avec un acide.

Ces coupleurs peuvent notamment être choisis parmi le 2-méthyl-5-amino-phénol, le 5-N-(β-hydroxyéthyl)-amino-2-méthyl-phénol, le 3-amino-phénol, le 1,3-dihydroxy-benzène, le 1,3-dihydroxy-2-méthyl-benzène, le 4-chloro-1,3-dihydroxy-benzène, le 2,4-diamino-1-(β-hydroxyéthyloxy)-benzène, le 2-amino-4-(β-hydroxyéthylamino)-1-méthoxy-benzène, le 1,3-diamino-benzène, le 1,3-bis-(2,4-diaminophénoxy)-propane, le sésamol, l'α-naphtol, le 6-hydroxy-indole, le 4-hydroxy-indole, le 4-hydroxy-N-méthyl-indole, la 6-hydroxy-indoline, la 2,6-dihydroxy-4-méthyl-pyridine, le 1-H-3-méthyl-pyrazole-5-one, le 1-phényl-3-méthyl-pyrazole-5-one, le 2,6-diméthyl-pyrazolo-[1,5-b]-1,2,4-triazole, le 2,6-diméthyl-[3,2-c]-1,2,4-triazole, le 6-méthyl-pyrazolo-[1,5-a]-benzimidazole, et leurs sels d'addition avec un acide.

Ces coupleurs représentent de préférence de 0,0001 à 10 % en poids environ du poids total de la composition tinctoriale prête à l'emploi et encore plus préférentiellement de 0,005 à 5 % en poids environ de ce poids.

D'une manière générale, les sels d'addition avec un acide utilisables dans le cadre des compositions tinctoriales de l'invention (bases d'oxydation et coupleurs) sont notamment choisis parmi les chlorhydrates, les bromhydrates, les sulfates et les tartrates, les lactates et les acétates.

La composition tinctoriale de l'invention peut encore contenir, en plus des colorants d'oxydation définis ci-dessus, des colorants directs pour enrichir les nuances en reflets. Ces colorants directs peuvent notamment alors être choisis parmi les colorants nitrés, azoïques ou anthraquinoniques.

L'invention a également pour objet un procédé de teinture des fibres kératiniques et en particulier des fibres kératiniques humaines telles que les cheveux mettant en oeuvre la composition tinctoriale prête à l'emploi telle que définie précédemment.

Selon ce procédé, on applique sur les fibres au moins une composition tinctoriale prête à l'emploi telle que définie précédemment, pendant un temps suffisant pour développer la coloration désirée, après quoi on rince, on lave éventuellement au shampooing, on rince à nouveau et on sèche.

Le temps nécessaire au développement de la coloration sur les fibres kératiniques est généralement compris entre 3 et 60 minutes et encore plus précisément 5 et 40 minutes.

Selon une forme de réalisation particulière de l'invention, le procédé comporte une étape préliminaire consistant à stocker sous forme séparée, d'une part, une composition (A) comprenant, dans un milieu approprié pour la teinture, au moins un colorant d'oxydation tel que défini précédemment et, d'autre part, une composition (B) renfermant, dans un milieu approprié pour la teinture, au moins une enzyme de type laccase et au moins un solvant choisi parmi les polyols ou leurs éthers ou polyéthers tel que défini précédemment, puis à procéder à leur mélange au moment de l'emploi avant d'appliquer ce mélange sur les fibres kératiniques.

Selon une forme de réalisation particulière de l'invention, le solvant [polyol(s) ou leurs éthers ou polyéthers] peut être incorporé dans la composition (A).

Un autre objet de l'invention est un dispositif à plusieurs compartiments ou "kit" de teinture ou tout autre système de conditionnement à plusieurs compartiments dont un premier compartiment renferme la composition (A) telle que définie ci-dessus et un second compartiment renferme la composition (B) telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Le milieu approprié pour les fibres kératiniques (ou support) des compositions tinctoriales prêtes à l'emploi des fibres kératiniques conformes à l'invention est généralement constitué par un mélange d'eau et d'au moins un polyol ou un éther ou polyéther de celui-ci, tel que défini ci-dessus. Il peut contenir en outre un ou des solvants organiques différents des polyols ou leurs éthers ou polyéthers utilisés conformément à l'invention, pour solubiliser les composés qui ne seraient pas suffisamment solubles dans le milieu. A titre de solvant organique, on peut par exemple citer les alcanols en C₁-C₄, tels que l'éthanol et l'isopropanol ainsi que les alcools aromatiques comme l'alcool benzylique, les produits analogues et leurs mélanges.

Lesdits solvants organiques peuvent être présents dans des proportions de préférence comprises entre 0,5 et 40 % en poids environ par rapport au poids total de la composition tinctoriale, et encore plus préférentiellement entre 1 et 20 % en poids environ.

Le pH des compositions tinctoriales prêtes à l'emploi des fibres kératiniques conformes à l'invention est choisi de telle manière que l'activité enzymatique de la laccase ne soit pas altérée. Il varie généralement de 4 à 11 environ, et plus préférentiellement de 6 à 9 environ.

Les compositions tinctoriales prêtes à l'emploi des fibres kératiniques conformes à l'invention peuvent également renfermer divers adjuvants utilisés classiquement dans les compositions pour la teinture des cheveux, tels que des agents tensioactifs anioniques, cationiques, non-ioniques, amphotères, zwittérioniques ou leurs mélanges, des polymères, des agents épaississants, des agents antioxydants, des enzymes différentes des laccases utilisées conformément à l'invention telles que par exemple des peroxydases ou des oxydo-réductases à 2 électrons, des agents de pénétration, des agents séquestrants, des parfums, des tampons, des agents dispersants, des agents filmogènes, des agents filtrants, des vitamines, des agents conservateurs, des agents opacifiants.

Bien entendu, l'homme de l'art veillera à choisir ce ou ces éventuels composés complémentaires de manière telle que les propriétés avantageuses attachées intrinsèquement aux compositions conformes à l'invention ne soient pas, ou substantiellement pas, altérées par la ou les adjonctions envisagées.

Les compositions tinctoriales prêtes à l'emploi des fibres kératiniques conformes à l'invention, peuvent se présenter sous des formes diverses, telles que sous forme de liquides, de crèmes, de gels, éventuellement pressurisés, ou sous toute autre forme appropriée pour réaliser une teinture des fibres kératiniques, et notamment des cheveux humains.

Dans les compositions tinctoriales de l'invention, le ou les colorants d'oxydation et la ou les laccases sont présents au sein de ladite composition qui doit être exempte d'oxygène gazeux, de manière à éviter toute oxydation prématurée du ou des colorants d'oxydation.

Des exemples concrets illustrant l'invention vont maintenant être donnés.

Dans ce qui suit ou ce qui précède, sauf mention contraire, les pourcentages sont exprimés en poids.

Les exemples suivants illustrent l'invention sans présenter un caractère limitatif.

### EXEMPLES 1 et 2 de TEINTURE

On a préparé les compositions tinctoriales prêtes à l'emploi suivante (teneurs en grammes) :

| **COMPOSITION** | **1** | **2** |
|---|---|---|
| Laccase issue de Rhus vernicifera à 180 unités/mg | | |
| vendue par la société SIGMA | 1,8 | 1,8 |
| Paraphénylènediamine | 0,254 | 0,254 |
| 2,4-diaminophénoxyéthanol, 2HCl | 0,260 | 0,260 |
| Tensio-actif non-ionique : alkylpolyglucoside en solution aqueuse à 60% de matière active (M.A.) | | |
| vendu sous la dénomination ORAMIX CG110 par la | 4,800 | 4,800 |
| société SEPPIC | (M.A.) | (M.A.) |
| Polyol selon l'invention | 20 | 20 |
| Agent de pH qs pH | 6,5 | 6,5 |
| Eau déminéralisée qsp | 100 | 100 |

| | | |
|---|---|---|
| Composition (1) : 1,4-butanediol. | | |
| Composition (2) : monobutyléther de propylèneglycol. | | |

Les compositions tinctoriales prêtes à l'emploi décrites ci-dessus ont été appliquées à la température de 30°C, sur des mèches de cheveux gris naturels à 90% de blancs pendant 40 minutes. Les cheveux ont ensuite été rincés, lavés avec un shampooing standard, puis séchés. Les cheveux ont été teints dans les deux cas en gris bleuté.

Dans les exemples décrits ci-dessus, 1,8% de Rhus vernicifera laccase à 180 unités/mg peut être remplacé par 1% de Pyricularia Orizae laccase à 100unités/mg vendu par la société I.C.N.

## Revendications

1. Composition prête à l'emploi pour la teinture des fibres kératiniques et en particulier des fibres kératiniques humaines telles que les cheveux, **caractérisée par le fait qu'**elle comprend, dans un support approprié pour la teinture des fibres kératiniques :
(a) au moins une enzyme du type laccase ;
(b) au moins un solvant choisi parmi
- les αω-diols ramifiés ou non ramifiés en C₃-C₈ ;
- les diols 1,2 - 1,4 - 1,5 - 2,3 - 2,4 - 2,5 - 2,6 - 2,7 - 2,8 - 3,4 - 3,5 - 3,6 en C₅-C₈ ramifiés ou non ramifiés ;
- les diols 1,3 en C₆-C₈ ramifiés ou non ramifiés ;
- les triols en C₄-C₈ ;
- le diéthylène glycol et le dipropylène glycol ;
- les monoalkyléthers en C₃-C₈ de glycols en C₃-C₉ ; monoalkyléthers C₅-C₉ d'éthylène glycol ; monoalkytéthers en C₁-C₂ de glycols en C₅-C₉ ; monoalkyléthers en C₂ des 1,2 - 1,3 - 1,4 - 2,3- butanediols ; les monoalkyléthers de glycols à nombre total d'atomes de carbone égal à 5 ;
- les dialkyléthers en C₁-C₉ de glycols en C₂-C₉ ;
- les phényl éthers de glycols en C₃-C₉ ;
(c) au moins un colorant d'oxydation,
le pH de la composition variant de 4 à 11.

2. Composition selon la revendication 1, **caractérisée par le fait que** la ou les laccases sont choisies parmi les laccases d'origine végétale, d'origine animale, d'origine fongique, d'origine bactérienne, ou obtenues par biotechnologie.

3. Composition selon l'une quelconque des revendications 1 à 2, où les laccases sont choisies parmi celles produites par des végétaux effectuant la synthèse chlorophyllienne.

4. Composition selon la revendication 3, où les laccases sont choisies parmi celles extraites des Anacardiacées ou des Podocarpacées , de Rosmarinus off., de Solanum tuberosum, d'Iris sp., de Coffea sp., de Daucus carrota, de Vinca minor, Persea americana, Catharenthus roseus, Musa sp., Malus pumila, Gingko biloba, Monotropa hypopithys (sucepin), Aesculus sp., Acer pseudoplatanus, Prunus persica, Pistacia palaestina.

5. Composition selon la revendication 2, où les laccases sont choisies parmi celles issues de Pyricularia orizae, de Polyporus versicolor, de Rhizoctonia praticola, de Rhus vemicifera, de Scytalidium, de Polyporus pinsitus, de Myceliophtora thermophila, de Rhizoctonia solani, de Tramates versicolor, de Fomes fomentarius, de Chaetomium thermophile, de Neurospora crassa, de Coriolus versicol, de Botrytis cinerea, de Rigidoporus lignosus, de Phellinus noxius, de Pleurotus ostreatus, d'Aspergillus nidulans, de Podospora anserina, d'Agaricus bisporus, de Ganoderma lucidum, de Glomerella cingulata, de Lactarius piperatus, de Russula delica, d'Heterobasidion annosum, de Thelephora terrestris, de Cladosporium cladosporioides, de Cerrena unicolor, de Coriolus hirsutus, de Ceriporiopsis subvermispora, de Coprinus cinereus, de Panaeolus papilionaceus, de Panaeolus sphinctrinus, de Schizophyllum commune, de Dichomitius squalens, ainsi que leurs variantes.

6. Composition selon l'une quelconque des revendications 1 à 5, **caractérisée par le fait que** la ou les laccases sont présentes dans des quantités allant de 0,5 à 2000 lacu, ou de 1000 à 4.10⁷ unités u, ou de 20 à 2.10⁸ unités ulac, pour 100g de composition.

7. Composition selon l'une quelconque des revendications 1 à 6, **caractérisée par le fait que** le solvant est choisi parmi :
- les αω-diols ramifiés ou non ramifiés en C₃-C₈ ;
- le diéthylène glycol et le dipropylène glycol ;
- les monoalkyléthers en C₃-C₈ de glycols en C₃-C₉ ;
- les monoalkyléthers en C₁-C₂ de glycols en C₅-C₉.

8. Composition selon l'une quelconque des revendications 1 à 7, **caractérisée par le fait que** le solvant selon l'invention est présent dans des proportions allant de 0,1 à 40% en poids, et de préférence de 0,5 à 20% en poids par rapport au poids total de la composition.

9. Composition selon l'une quelconque des revendications précédentes, **caractérisée par le fait que** le ou les colorants d'oxydation sont choisis parmi les bases d'oxydation et/ou les coupleurs.

10. Composition selon la revendication 9, **caractérisée par le fait que** les bases d'oxydation sont choisis parmi les ortho- ou para- phénylènediamines, les bis-phénylalkylènediamines, les ortho- ou para- aminophénols, et les bases hétérocycliques, ainsi que les sels d'addition de ces composés avec un acide.

11. Composition selon la revendication 9 ou 10, **caractérisée par le fait que** les bases d'oxydation sont présentes dans des concentrations allant de 0,0005 à 12% en poids par rapport au poids total de la composition.

12. Composition selon la revendication 9, **caractérisée par le fait que** les coupleurs sont choisis parmi les métaphénylènediamines, les métaaminophénols, les métadiphénols, les coupleurs hétérocycliques, et les sels d'addition de ces composés avec un acide.

13. Composition selon la revendication 9 ou 12, **caractérisée par le fait que** les coupleurs sont présents dans des concentrations allant de 0,0001 à 10% en poids par rapport au poids total de la composition.

14. Composition selon l'une quelconque des revendications 9 à 13, **caractérisée par le fait que** les sels d'addition avec un acide des bases d'oxydation et des coupleurs sont choisis parmi les chlorhydrates, les bromhydrates, les sulfates, les tartrates, les lactates et les acétates.

15. Composition selon l'une quelconque des revendications précédentes, **caractérisée par le fait qu'**elle contient en outre des colorants directs.

16. Composition selon l'une quelconque des revendications précédentes, **caractérisée par le fait que** le milieu approprié pour les fibres kératiniques (ou support) est constitué par un mélange d'eau et d'au moins un solvant choisi parmi ceux définis dans les revendications 1 et 7.

17. Composition selon la revendication 16, **caractérisée par le fait que** le milieu comprend en outre au moins un solvant organique.

18. Composition selon l'une quelconque des revendications précédentes, **caractérisée par le fait que** le pH varie de 6 à 9.

19. Composition selon l'une quelconque des revendications précédentes, **caractérisée par le fait qu'**elle contient en plus au moins un adjuvant cosmétique utilisé classiquement dans les compositions pour la teinture des cheveux,choisi dans le groupe constitué par des agents tensio-actifs, des polymères, des agents épaississants, des agents antioxydants, des enzymes différentes des laccases, des agents de pénétration, des agents séquestrants, des parfums, des tampons, des agents dispersants, des agents filmogènes, des agents filtrants, des vitamines, des agents conservateurs, des agents opacifiants.

20. Procédé de teinture des fibres kératiniques et en particulier des fibres kératiniques humaines telles que les cheveux, **caractérisé par le fait qu'**on applique sur lesdites fibres au moins une composition tinctoriale prête à l'emploi telle que définie dans l'une quelconque des revendications précédentes, pendant un temps suffisant pour développer la coloration désirée.

21. Procédé selon la revendication 20, **caractérisé par le fait qu'**il comporte une étape préliminaire consistant à stocker sous forme séparée, d'une part, une composition (A) comprenant, dans un milieu approprié pour la teinture, au moins un colorant d'oxydation tel que défini dans l'une quelconque des revendications 9 à 14 et d'autre part, une composition (B) renfermant, dans un milieu approprié pour les fibres kératiniques, au moins une enzyme du type laccase telle que définie dans l'une quelconque des revendications 1 à 6 puis à procéder à leur mélange au moment de l'emploi avant d'appliquer ce mélange sur les fibres kératiniques ; la composition (A) ou la composition (B) contenant le solvant tel que défini dans les revendications 1 et 7.

22. Dispositif à plusieurs compartiments ou "kit" de teinture, **caractérisé par le fait qu'**il comporte un premier compartiment renfermant la composition (A) telle que définie dans la revendication 21 et un second compartiment renfermant la composition (B) telle que définie dans la revendication 21; la composition (A) ou la composition (B) contenant le solvant tel que défini dans l'une des revendications 1 et 7.

## Patentansprüche

1. Gebrauchsfertige Zusammensetzung zum Färben von Keratinfasern, insbesondere menschlichen Keratinfasern, wie zum Färben der Haare, die in einem zum Färben von Keratinfasern geeigneten Träger enthält:
(a) mindestens ein Enzym vom Laccase-Typ;
(b) mindestens ein Lösungsmittel, das ausgewählt ist unter:
- verzweigten oder nicht verzweigten αω-Diolen mit 3 bis 8 Kohlenstoffatomen;
- verzweigten oder nicht verzweigten 1,2 - 1,4 - 1,5 - 2,3 - 2,4 - 2,5 - 2,6 - 2,7 - 2,8 - 3,4 - 3,5 - 3,6 -Diolen mit 5 bis 8 Kohlenstoffatomen;
- verzweigten oder nicht verzweigten 1,3-Diolen mit 6 bis 8 Kohlenstoffatomen;
- Triolen mit 4 bis 8 Kohlenstoffatomen;
- Diethylenglykol und Dipropylenglykol;
- C₃₋₈-Monoalkylethern von C₃₋₉-Glykolen; C₅₋₉-Monoalkylethern von Ethylenglykol; C₁₋₂-Monoalkylethern von C₅₋₉-Glykolen; C₂-Monoalkylethern von 1,2 - 1,3 - 1,4 - 2,3 -Butandiolen; Monoalkylethern von Glykolen mit einer Gesamtzahl der Kohlenstoffatome von 5;
- C₁₋₉-Dialkylethern von C₂₋₉-Glykolen; und
- Phenylethern von C₃₋₉-Glykolen;
(c) mindestens einen Oxidationsfarbstoff,
wobei der pH-Wert der Zusammensetzung im Bereich von 4 bis 11 liegt.

2. Zusammensetzung nach Anspruch 1, **dadurch gekennzeichnet, dass** die Laccase(n) unter den Laccasen pflanzlicher Herkunft, den Laccasen tierischer Herkunft, den Laccasen, die von Pilzen gebildet werden, den Laccasen bakterieller Herkunft oder den biotechnologisch hergestellten Laccasen ausgewählt sind.

3. Zusammensetzung nach einem der Ansprüche 1 oder 2, wobei die Laccasen unter den Laccasen ausgewählt sind, die von Pflanzen gebildet werden, die Chlorophyll synthetisieren.

4. Zusammensetzung nach Anspruch 3, wobei die Laccasen unter den Laccasen ausgewählt sind, die aus Anacardiaceae oder Podocarpaceae, Rosmarinus off., Solanum tuberosum, Iris sp., Coffea sp., Daucus carrota, Vinca minor, Persea americana, Catharenthus roseus, Musa sp., Malus pumila, Gingko biloba, Monotropa hypopithys (Fichtenspargel), Aesculus sp., Acer pseudoplatanus, Prunus persica oder Pistacia palaestina extrahiert wurden.

5. Zusammensetzung nach Anspruch 2, wobei die Laccasen unter den Laccasen ausgewählt sind, die von Pyricularia oryzae, Polyporus versicolor, Rhizoctonia praticola, Rhus vernicifera, Scytalidium, Polyporus pinsitus, Myceliophtora thermophila, Rhizoctonia solani, Tramates versicolor, Fomes fomentarius, Chaetomium thermophile, Neurospora crassa, Coriolus versicol, Botrytis cinerea, Rigidoporus lignosus, Phellinus noxius, Pleurotus ostreatus, Aspergillus nidulans, Podospora anserina, Agaricus bisporus, Ganoderma lucidum, Glomerella cingulata, Lactarius piperatus, Russula delica, Heterobasidion annosum, Thelephora terrestris, Cladosporium cladosporioides, Cerrena unicolor, Coriolus hirsutus, Ceriporiopsis subvermispora, Coprinus cinereus, Panaeolus papilionaceus, Panaeolus sphinctrinus, Schizophyllum commune, Dichomitus squalens und deren Varianten stammen.

6. Zusammensetzung nach einem der Ansprüche 1 bis 5, **dadurch gekennzeichnet, dass** die Laccase(n) in einem Mengenanteil im Bereich von 0,5 bis 2000 lacu oder 1000 bis 4·10⁷ Einheiten u oder 20 bis 2·10⁶ Einheiten ulac pro 100 g Zusammensetzung vorliegen.

7. Zusammensetzung nach einem der Ansprüche 1 bis 6, **dadurch gekennzeichnet, dass** das Lösungsmittel ausgewählt ist unter:
- verzweigten oder nicht verzweigten αω-Diolen mit 3 bis 8 Kohlenstoffatomen;
- Diethylenglykol und Dipropylenglykol;
- C₃₋₈-Monoalkylethern von C₃₋₉-Glykolen;
- C₁₋₂-Monoalkylethern von C₅₋₉-Glykolen.

8. Zusammensetzung nach einem der Ansprüche 1 bis 7, **dadurch gekennzeichnet, dass** das erfindungsgemäße Lösungsmittel in einem Mengenanteil von 0,1 bis 40 Gew.-% und vorzugsweise 0,5 bis 20 Gew.-%, bezogen auf das Gesamtgewicht der Zusammensetzung, enthalten ist.

9. Zusammensetzung nach einem der vorhergehenden Ansprüche, **dadurch gekennzeichnet, dass** der oder die Oxidationsfarbstoff(e) unter den Oxidationsbasen und/oder Kupplern ausgewählt sind.

10. Zusammensetzung nach Anspruch 9, **dadurch gekennzeichnet, dass** die Oxidationsbasen unter den o- oder p-Phenylendiaminen, Bisphenylalkylendiaminen, o- oder p-Aminophenolen und den heterocyclischen Basen sowie den Additionssalzen dieser Verbindungen mit einer Säure ausgewählt sind.

11. Zusammensetzung nach Anspruch 9 oder 10, **dadurch gekennzeichnet, dass** die Oxidationsbasen in Konzentrationen von 0,0005 bis 12 Gew.-%, bezogen auf das Gesamtgewicht der Zusammensetzung, vorliegen.

12. Zusammensetzung nach Anspruch 9, **dadurch gekennzeichnet, dass** die Kuppler unter den m-Phenylendiaminen, m-Aminophenolen, m-Dihydroxybenzolen und heterocyclischen Kupplern und deren Additionssalzen mit einer Säure ausgewählt sind.

13. Zusammensetzung nach Anspruch 9 oder 12, **dadurch gekennzeichnet, dass** die Kuppler in Konzentrationen von 0,0001 bis 10 Gew.-%, bezogen auf das Gesamtgewicht der Zusammensetzung, vorliegen.

14. Zusammensetzung nach einem der Ansprüche 9 bis 13, **dadurch gekennzeichnet, dass** die Additionssalze der Oxidationsbasen und Kuppler mit einer Säure unter den Hydrochloriden, Hydrobromiden, Sulfaten, Tartraten, Lactaten und Acetaten ausgewählt sind.

15. Zusammensetzung nach einem der vorhergehenden Ansprüche, **dadurch gekennzeichnet, dass** sie ferner Direktfarbstoffe enthält.

16. Zusammensetzung nach einem der vorhergehenden Ansprüche, **dadurch gekennzeichnet, dass** das für Keratinfasern geeignete Medium (oder der Träger) aus einem Gemisch von Wasser und mindestens einem Lösungsmittel besteht, das unter den in den Ansprüchen 1 und 7 definierten Lösungsmitteln ausgewählt ist.

17. Zusammensetzung nach Anspruch 16,**dadurch gekennzeichnet, dass** das das Medium ferner mindestens ein organisches Lösungsmittel enthält.

18. Zusammensetzung nach einem der vorhergehenden Ansprüche, **dadurch gekennzeichnet, dass** der pH-Wert im Bereich von 6 bis 9 liegt.

19. Zusammensetzung nach einem der vorhergehenden Ansprüche, **dadurch gekennzeichnet, dass** sie ferner mindestens einen herkömmlich in Zusammensetzungen zum Färben der Haare verwendeten kosmetischen Zusatzstoff enthält, der unter den grenzflächenaktiven Stoffen, Polymeren, Verdickungsmitteln, Antioxidantien, Enzymen, die von Laccasen verschieden sind, Penetrationsmitteln, Maskierungsmitteln, Parfums, Puffern, Dispergiermitteln, Filmbildnern, Filtern, Vitaminen, Konservierungsmitteln und Trübungsmitteln ausgewählt ist.

20. Verfahren zum Färben von Keratinfasern und insbesondere menschlichen Keratinfasern, wie Haaren, **dadurch gekennzeichnet, dass** auf die Fasern während einer Zeitspanne, die zur Entwicklung der gewünschten Färbung ausreichend ist, mindestens eine gebrauchsfertige Farbmittelzusammensetzung nach einem der vorhergehenden Ansprüche aufgebracht wird.

21. Verfahren nach Anspruch 20, **dadurch gekennzeichnet, dass** es einen vorbereitenden Schritt umfasst, der darin besteht, einerseits eine Zusammensetzung (A), die in einem zum Färben geeigneten Medium mindestens einen Oxidationsfarbstoff nach einem der Ansprüche 9 bis 14 enthält, und andererseits eine Zusammensetzung (B) getrennt voneinander aufzubewahren, die in einem für Keratinfasern geeigneten Medium mindestens ein Enzym vom Laccase-Typ nach einem der Ansprüche 1 bis 6 enthält, und diese bei der Anwendung zu vermischen, bevor das Gemisch auf die Keratinfasern aufgebracht wird, wobei die Zusammensetzung (A) oder die Zusammensetzung (B) ein Lösungsmittel nach einem der Ansprüche 1 und 7 enthält.

22. Vorrichtung mit mehreren Abteilungen oder "Kit" zum Färben, **dadurch gekennzeichnet, dass** sie eine erste Abteilung mit der in Anspruch 21 definierten Zusammensetzung (A) und eine zweite Abteilung mit der in Anspruch 21 definierten Zusammensetzung (B) enthält, wobei die Zusammensetzung (A) oder die Zusammensetzung (B) ein Lösungsmittel nach einem der Ansprüche 1 und 7 enthält.

## Claims

1. Ready-to-use composition for dyeing keratinous fibres, and in particular human keratinous fibres such as hair, **characterized in that** it comprises, in a carrier appropriate for dyeing keratinous fibres:
(a) at least one enzyme of the laccase type;
(b) at least one solvent chosen from
- branched or unbranched C₃-C₈ αω-diols;
- branched or unbranched C₅-C₈ 1, 2 - 1, 4 - 1, 5 - 2,3 - 2,4 - 2, 5 - 2, 6 - 2, 7 - 2, 8 - 3, 4 - 3, 5 - 3,6 diols;
- branched or unbranched C₆-C₈ 1,3 diols;
- C₄-C₈ triols;
- diethylene glycol and dipropylene glycol;
- C₃-C₈ monoalkyl ethers of C₃-C₉ glycols; C₅-C₉ monoalkyl ethers of ethylene glycol; C₁-C₂ monoalkyl ethers of C₅-C₉ glycols; C₂ monoalkyl ethers of 1,2 - 1,3 - 1,4 - 2,3- butanediols; monoalkyl ethers of glycols containing a total number of carbon atoms equal to 5;
- C₁-C₉ dialkyl ethers of C₂-C₉ glycols;
- phenyl ethers of C₃-C₉ glycols;
(c) at least one oxidation dye;
the pH of the composition varying from 4 to 11.

2. Composition according to Claim 1, **characterized in that** the laccase(s) are chosen from laccases of plant origin, animal origin, fungal origin, bacterial origin or are obtained by biotechnology.

3. Composition according to either of Claims 1 and 2, where the laccases are chosen from those produced by plants performing chlorophyll synthesis.

4. Composition according to Claim 3, where the laccases are chosen from those extracted from Anacardiaceae or Podocarpaceae, Rosmarinus off., Solanum tuberosum, Iris sp., Coffea sp., Daucus carrota, Vinca minor, Persea americana, Catharenthus roseus, Musa sp., Malus pumila, Gingko biloba, Monotropa hypopithys (Indian pipe), Aesculus sp., Acer pseudoplatanus, Prunus persica, Pistacia palaestina.

5. Composition according to Claim 2, where the laccases are chosen from those derived from Pyricularia orizae, Polyporus versicolor, Rhizoctonia praticola, Rhus vernicifera, Scytalidium, Polyporus pinsitus, Myceliophtora thermophila, Rhizoctonia solani, Tramates versicolor, Fomes fomentarius, Chaetomium thermophile, Neurospora crassa, Coriolus versicol, Botrytis cinerea, Rigidoporus lignosus, Phellinus noxius, Pleurotus ostreatus, Aspergillus nidulans, Podospora anserina, Agaricus bisporus, Ganoderma lucidum, Glomerella cingulata, Lactarius piperatus, Russula delica, Heterobasidion annosum, Thelephora terrestris, Cladosporium cladosporioides, Cerrena unicolor, Coriolus hirsutus, Ceriporiopsis subvermispora, Coprinus cinereus, Panaeolus papilionaceus, Panaeolus sphinctrinus, Schizophyllum commune, Dichomitius squalens and variants thereof.

6. Composition according to any one of Claims 1 to 5, **characterized in that** the laccase(s) are provided in quantities ranging from 0.5 to 2000 lacu, or from 1000 to 4×10⁷ u units, or from 20 to 2×10⁶ lacu units, per 100 g of composition.

7. Composition according to any one of Claims 1 to 6, **characterized in that** the solvent is chosen from:
- branched or unbranched C₃-C₈ αω-diols;
- diethylene glycol and dipropylene glycol;
- C₃-C₈ monoalkyl ethers of C₃-C₉ glycols;
- C₁-C₂ monoalkyl ethers of C₅-C₉ glycols.

8. Composition according to any one of Claims 1 to 7, **characterized in that** the solvent according to the invention is present in proportions ranging from 0.1 to 40% by weight, and preferably from 0.5 to 20% by weight relative to the total weight of the composition.

9. Composition according to any one of the preceding claims, **characterized in that** the oxidation dye(s) are chosen from oxidation bases and/or couplers.

10. Composition according to Claim 9, **characterized in that** the oxidation bases are chosen from ortho- or para-phenylenediamines, bisphenylalkylenediamines, ortho- or para-aminophenols, and heterocylic bases, as well as the addition salts of these compounds with an acid.

11. Composition according to Claim 9 or 10, **characterized in that** the oxidation bases are present in concentrations ranging from 0.0005 to 12% by weight relative to the total weight of the composition.

12. Composition according to Claim 9, **characterized in that** the couplers are chosen from meta-phenylenediamines, meta-aminophenols, meta-diphenols, heterocyclic couplers, and the addition salts of these compounds with an acid.

13. Composition according to Claim 9 or 12, **characterized in that** the couplers are present in concentrations ranging from 0.0001 to 10% by weight relative to the total weight of the composition.

14. Composition according to any one of Claims 9 to 13, **characterized in that** the addition salts with an acid of the oxidation bases and couplers are chosen from hydrochlorides, hydrobromides, sulphates, tartrates, lactates and acetates.

15. Composition according to any one of the preceding claims, **characterized in that** it contains, in addition, direct dyes.

16. Composition according to any one of the preceding claims, **characterized in that** the medium appropriate for keratinous fibres (or carrier) consists of a mixture of water and of at least one solvent chosen from those defined in Claims 1 and 7.

17. Composition according to Claim 16, **characterized in that** the medium comprises, in addition, at least one organic solvent.

18. Composition according to any one of the preceding claims, **characterized in that** the pH varies from 6 to 9.

19. Composition according to any one of the preceding claims, **characterized in that** it contains, in addition, at least one cosmetic adjuvant conventionally used in hair-dyeing compositions, chosen from the group consisting of surfactants, polymers, thickening agents, antioxidants, enzymes different from laccases, penetrating agents, sequestering agents, perfumes, buffers, dispersing agents, film-forming agents, screening agents, vitamins, preservatives or opacifying agents.

20. Method of dyeing keratinous fibres, and in particular human keratinous fibres such as hair, **characterized in that** at least one ready-to-use dyeing composition as defined in any one of the preceding claims is applied to the said fibres for a sufficient time to develop the desired colour.

21. Method according to Claim 20, **characterized in that** it comprises a preliminary step consisting in storing in a separate form, on the one hand, a composition (A) comprising, in a medium appropriate for dyeing, at least one oxidation dye as defined in any one of claims 9 to 14, and, on the other hand, a composition (B) containing, in a medium appropriate for keratinous fibres, at least one enzyme of the laccase type as defined in any one of Claims 1 to 6, and then in mixing them at the time of use before applying this mixture to the keratinous fibres; the composition (A) or the composition (B) containing the solvent as defined in Claims 1 and 7.

22. Multicompartment device or dyeing "kit", **characterized in that** it comprises a first compartment containing the composition (A) as defined in Claim 21 and a second compartment containing the composition (B) as defined in Claim 21; the composition (A) or the composition (B) containing the solvent as defined in one of Claims 1 and 7.
